# EUROPEAN PATENT SPECIFICATION

(11) **EP 2 953 583 B1**
(45) Date of publication and mention of the grant of the patent: **23.08.2017**
(21) Application number: 14706397.8
(22) Date of filing: 05.02.2014
(51) Int. Cl.: A61F 2/30, A61F 2/38

(54) **TAPERED TIBIAL AUGMENT**
KONISCHE WADENBEINVERSTÄRKUNG
AUGMENTATION TIBIALE CONIQUE

(30) Priority: 07.02.2013 US 201361762040 P; 15.03.2013 US 201361789245 P
(43) Date of publication of application: 16.12.2015
(73) Proprietor: Zimmer, Inc., Warsaw, Indiana 46580 (US)
(72) Inventor: METZGER, Dianne, S., Huntington, IN 46750 (US); VAUGHAN, Ian, R., Fort Wayne, IN 46814 (US); GREY, Calie, B., Warsaw, IN 46582 (US)
(74) Representative: Mays, Julie
(86) International application number: PCT/US2014/014834
(87) International publication number: WO 2014/123979

(56) References cited:
- US-A- 5 152 797
- US-A- 5 344 461
- US-A- 5 387 241
- US-A1- 2012 310 361

## Description

### BACKGROUND

A human knee is a joint that connects a femur to a tibia (sometimes referred to as the thigh bone and the shin bone, respectively). The knee allows for pivoting between the femur and the tibia. The pivoting has a pivot axis aligned with the medial-lateral direction. Some types of injury, disease, or degeneration can produce pain and/or restricted motion in the knee joint. One treatment for certain types of damage to a knee joint is surgery. For relatively mild knee damage, the knee may be repaired. For more severe damage, the knee may be replaced.

In total knee replacement surgery, all of the articulating elements within the knee joint are replaced. During the surgery, a distal end (sometimes referred to as an inferior end or a bottom end) of the femur is cut to a particular shape, and then a femoral implant is attached to the cut distal end of the femur. The femoral implant typically includes a pair of convex condylar surfaces. The condylar surfaces are shaped to slide within corresponding concave bearing indentations on a tibial bearing surface. The tibial bearing surface is typically formed from a hard plastic, which allows the condylar surfaces to slide in the indentations with reduced friction.

The tibial bearing surface is typically attached to a proximal side (sometimes referred to as a superior side or a top side) of a tibial platform. The tibial platform can include a tibial stem coupled to its distal side. During surgery, a proximal end of the tibia is cut to a particular size or shape, and then the tibial stem is attached to the proximal end of the tibia.

In some surgical cases, there has been a loss of bone at the proximal portion of the tibia. In order to compensate for the missing bone, a tibial augment can be implanted. Tibial augments can be attached between a distal side of the tibial platform and the proximal end of the tibia. US2012/3100361 discloses a tibial support structure, UD5344461 discloses a modular augmentation block, US5387241 discloses a prosthetic joint implant, and US 5152797 discloses a modular prosthesis system.

### OVERVIEW

In typical knee repair or replacement surgery, a femoral implant includes a pair of convex condylar surfaces that can slide within corresponding concave bearing indentations on a tibial bearing surface. The tibial bearing surface is disposed on a proximal side of a tibial platform. A stem extends distally from the tibial platform, and attaches to a cut proximal end of the tibia. A tibial augment can attach to the distal side of the tibial platform, and can reduce or eliminate a lateral overhang of the tibial platform with respect to the cut proximal end of the tibia. The tibial augment may have a tapered periphery. The taper may extend inward in a distal direction. The taper may vary along the periphery of the tibial augment, so that at least a portion of a medial taper is different from at least a portion of a lateral taper. Such a taper that varies along the periphery of the tibial augment may help reduce or eliminate the lateral overhang of the tibial platform with respect to the cut proximal end of the tibia, and may do so better than a taper that does not vary along the periphery of the tibial augment. The present invention is defined in claim 1 and is directed towards a device for augmenting a tibial stem for a human knee. The Detailed Description is included to provide further information about the present invention.

### BRIEF DESCRIPTION OF THE DRAWINGS

In the drawings, which are not necessarily drawn to scale, like numerals may describe similar components in different views. The drawings illustrate generally, by way of example, but not by way of limitation, various embodiments discussed in the present document.
FIG. 1 is a perspective drawing of an example tibial platform, an example tibial augment, and an example proximal end of a tibia. The elements shown are for a left knee, viewed upright and from behind.
FIG. 2 is a perspective drawing of an example tibial augment and an example tibial platform. The elements shown are for a right knee, viewed upside-down and from behind.
FIGS. 3-8 are schematic drawings of an example system of differently-sized tibial augments, shown superimposed on differently-sized tibia bone cross-sections. The elements shown are for tibial augments having a thickness of 10 mm.
FIGS. 9-14 are schematic drawings of an example system of differently-sized tibial augments, shown superimposed on differently-sized tibia bone cross-sections. The elements shown are for tibial augments having a thickness of 5 mm.

### DETAILED DESCRIPTION

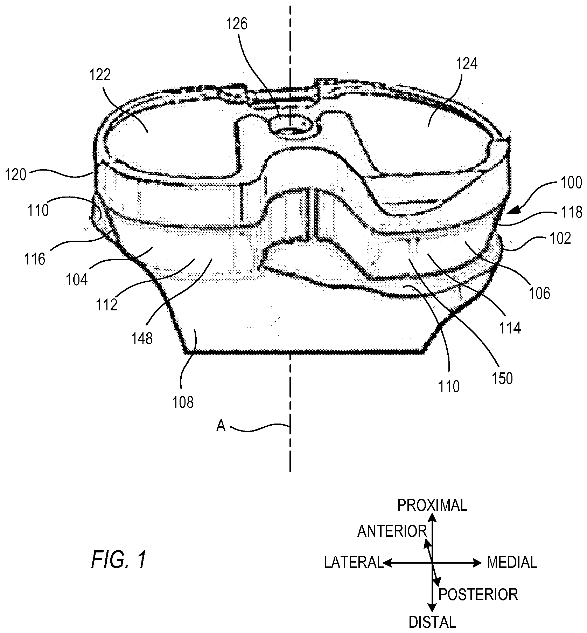

FIG. 1 is a perspective drawing of an example tibial platform 120, an example tibial augment 100, and an example proximal end of a tibia 108. The elements shown are for a left knee, viewed upright and from behind. It will be understood that similar elements may be used for a right knee, but with the features on the elements being flipped about a plane of symmetry that extends between lateral and medial halves of the knee.

During surgery, a proximal end of the tibia 108 can be cut to have a flat portion 110. The flat portion 110 may be oriented so that the tibial augment 100 and the tibial platform 120 may be parallel to the flat portion 110, and may remain in contact with the flat portion 110 after the surgery has been completed.

The tibial platform 120 may be generally planar, and may have a longitudinal axis (A) extending perpendicular to a plane of the tibial platform 120. The tibial platform has a footprint (i.e., a cross-section taken perpendicular to the longitudinal axis) that roughly matches a footprint 102 of the tibia bone at the flat portion 110. The tibial platform 120 may include features on its proximal side for supporting a tibial bearing surface. In some examples, the tibial bearing surface is a formed as a proximal side of a spacer (not shown). The tibial bearing surface may have one or more concave bearing indentations on its proximal side for articulation with one or more corresponding condylar surfaces on a femoral implant. The spacer may be formed from a hard plastic material that has a relatively low friction with the femoral implant. The tibial bearing surface may have a flat distal side, which may be cemented during surgery to a corresponding flat portion on the proximal side of the tibial platform 120. The proximal side of the tibial platform 120 may optionally include a ridge or other suitable features around its perimeter, which may help place the spacer during surgery, and may help protect the spacer during use after surgery. The example tibial platform 120 includes a hole 126 therethrough, which defines a longitudinal axis (A) for the tibial elements. The tibial platform 120 has a lateral side 122 and a medial side 124 on opposite sides of the longitudinal axis (A). The hole 126 may be used to secure a stem (not shown) extending distally from the tibial platform 120, and extending into the bone of the tibia 108.

For some examples, the proximal portion of the tibia 108 shows a loss of bone. In order to compensate for the missing bone, the tibial platform 120 and the tibia 108 may be spaced apart by a tibial augment 100. A tibial augment 100 is configured to attach to the tibial platform 120 at or near a distal side of the tibial platform 120. Although the tibial augment 100 may be formed as a single element, the tibial augment 100 is more commonly formed as two discrete elements, which include a medial side 106 and a lateral side 104. The medial 106 and lateral 104 sides 106 of the tibial augment 100 attach to the medial 124 and lateral 122 sides of the tibial platform 120, respectively. In some examples, the tibial augment 100 is an augment trial that attaches removably to the tibial platform 120, such as with one or more set screws. In other examples, the tibial augment 100 is an augment implant that attaches fixedly to the tibial platform 120, such as with cement.

The tibial augment 100 includes a taper on at least a portion of its periphery. Such a tapering may reduce or prevent a lateral overhang of the implanted tibial elements over the edge of the bone at the proximal end of the tibia 108. For instance, note that a footprint of the tibial augment 100 is smaller at its distal side than at its proximal side. Such a reduced lateral footprint helps ensure that that the tibial augment 100 does not hang over the edge of the bone at the proximal end of the tibia 108, or at least reduces the overhang to an acceptably small region. In the example of FIG. 1, a medial posterior portion 150 of the tibial augment 100 is tapered inward, so that a small region of the flat portion 110 of the bone extends radially outward beyond the medial posterior portion 150 of the tibial augment 100. Similarly, a small region of the flat portion 110 of the bone (at the leftmost edge of FIG. 1) extends readily outward beyond a lateral edge of the tibial augment 110. In general, it will be understood that the shape of the tibia bone varies from patient to patient, and that the specific bone shape shown in FIG. 1 is just an example, and should not be construed as limiting in any way.

The present inventors have found that having a taper that varies along its periphery may be further advantageous. Such a varying taper may more closely follow the footprint of the bone at the proximal end of the tibia 108, and may desirably reduce the amount of exposed bone or overhang over the edge of the bone. The taper has values that differ in different regions of the tibial augment 100. For instance, the medial side 106 of the tibial augment 100 has a medial taper 118 on at least a portion of its periphery, the lateral side 104 of the tibial augment 100 has a lateral taper 116 on at least a portion of its periphery, and at least a portion of the medial taper 118 is different from at least a portion of the lateral taper 116.

An example range of medial tapers 118 may be between thirteen degrees and eighteen degrees, inclusive, although other values of the medial taper 118 may also be used. An example range of lateral tapers 116 may be between ten degrees and eighteen degrees, inclusive, although other values of the lateral taper 116 may also be used. The angles for these example numerical ranges are formed between the longitudinal axis (A) and the periphery; the angle would be zero if the periphery were parallel to the longitudinal axis (A). According to the invention, the lateral taper 116 has a first value that is constant around the periphery of the lateral side 104 of the tibial augment 100, the medial taper 118 has a second value that is constant around the periphery of the medial side 106 of the tibial augment 100, and the first and second values are different.

In other examples, the lateral taper 116 may vary around the periphery of the lateral side 104 of the tibial augment 100, and/or the medial taper 118 may vary around the periphery of the medial side 106 of the tibial augment 100. For instance, the lateral side 104 of the tibial augment 100 may include a lateral posterior portion 112, which has a lateral posterior taper 148 on at least a portion of its periphery. At least a portion of the lateral posterior taper 148 may be different from at least a portion of the lateral taper 116. Similarly, the medial side 106 of the tibial augment 100 may include a medial posterior portion 114, which has a medial posterior taper 150 on at least a portion of its periphery. At least a portion of the medial posterior taper 150 may be different from at least a portion of the medial taper 118. In most examples, the value of taper varies gradually, rather than discontinuously, in order to avoid sharp corners on the tibial augment 100. As such, there may be regions of the periphery that have a constant taper, and other regions in which the taper is continuously changing.

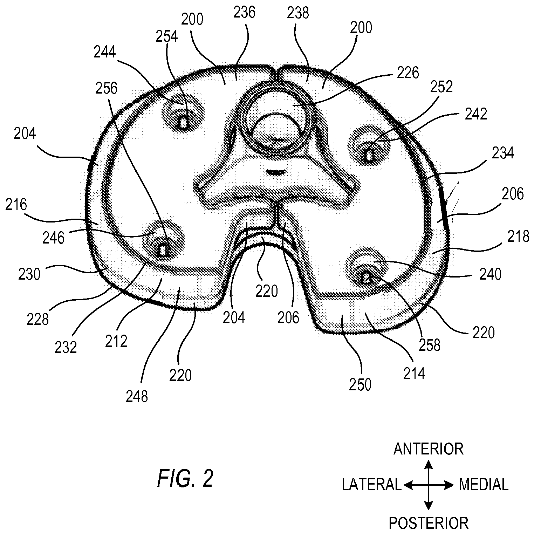

FIG. 2 is a perspective drawing of an example tibial augment 200 and an example tibial platform 220. The elements shown are for a right knee, viewed upside-down and from behind, as if looking upward from the point of view of the tibia. The distal side of the tibial augment 200 is more clearly shown in FIG. 2, along with the taper of the periphery of the tibial augment 200.

The tibial augment 200 includes a lateral side 204. The lateral side 204 includes a lateral taper 216 on at least a portion of its periphery. The lateral side 204 includes a lateral posterior portion 212. The lateral posterior portion 212 includes a lateral portion taper 248 on at least a portion of its periphery. The tibial augment 200 also includes a medial side 206, which, in this example, is separate from the lateral side 204. The lateral 204 and medial 206 sides are located on opposite sides of the hole 226 that can couple to a stem (not shown). The medial side 206 includes a medial taper 218 on at least a portion of its periphery. The medial side 206 includes a medial posterior portion 214. The medial posterior portion 214 includes a medial portion taper 250 on at least a portion of its periphery.

The tibial platform 220 has a periphery that is typically perpendicular to its proximal and distal sides, so that a footprint 228 of the proximal side of tibial platform 220 is the same size and shape as a footprint 230 of the distal side of tibial platform 220. The footprint of the proximal side of the tibial augment 200 is typically selected to match the footprint 230 of the distal side of tibial platform 220. The tibial augment 200 includes a lateral distal face 236, having a lateral distal footprint 232, and a medial distal face 238, having a medial distal footprint 234. When the tibial augment 200 is installed, the lateral distal footprint 232 and the medial distal footprint 234 are selected to coincide with or be slightly smaller than a footprint of the bone of the tibia.

The tibial augment 200 may include a plurality of augment holes 240, 242, 244, 246 extending longitudinally through the tibial augment 200. In the example of FIG. 2, four holes are shown, although other suitable numbers of augment holes can include two, three, five, six, or more than six. When the tibial augment 200 is attached to the tibial platform 220, the augment holes 240, 242, 244, 246 coincide with and are parallel to a respective plurality of platform holes 252, 254, 256, 258. The platform holes 252, 254, 256, 258 extend into a distal side of the tibial platform 220. In some examples, the platform holes 252, 254, 256, 258 do not extend fully though the tibial platform 220. In some examples, the platform holes 252, 254, 256, 258 are threaded, and the augment holes 240, 242, 244, 246 are unthreaded, so that the platform and augment holes may be used to securely screw the tibial augment 200 to the tibial platform 220.

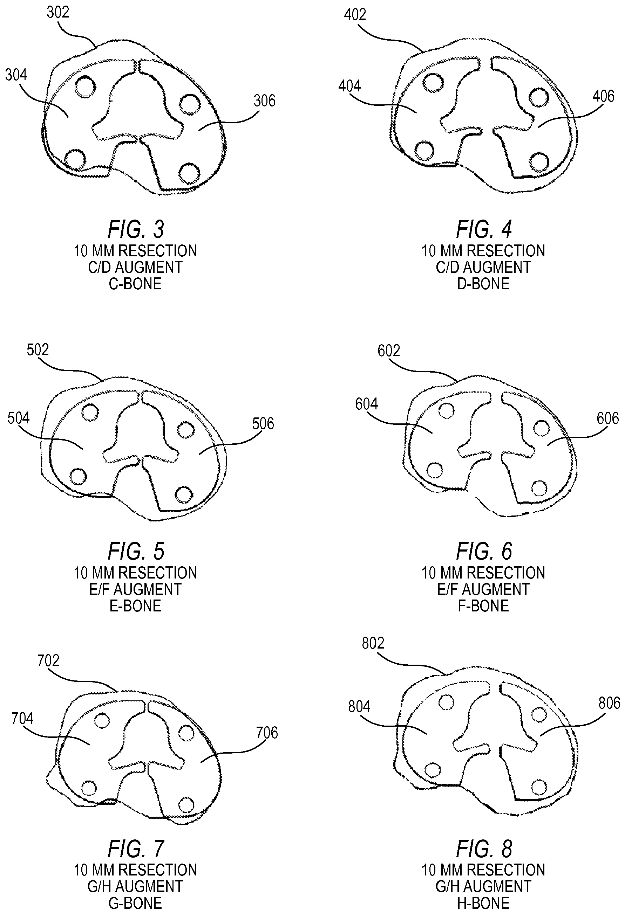

In some examples, the tibial augments are sold as systems. FIGS. 3-14 show an example configuration of a system that can span a range of bone sizes and resection values. In this particular example system, the tibial augments are available for two discrete values of resection, and three discrete augment sizes that can each accommodate a respective range of bone sizes. It will be understood that a system may include more or fewer numbers of parts. Using more parts in the system may be able to better fit a part to a particular set of patient conditions, but at the expense of maintaining inventory of more parts in the system, and possibly higher costs due to the increased number of part variations in the system.

Each of FIGS. 3-14 includes an end-on view of the distal side of a tibial augment. Each tibial augment has a lateral side, on the left-hand side of the figure, and has a medial side, on the right-hand side of the figure. In FIGS. 3-14, the lateral sides are denoted by element numbers ending in 04, and the medial sides are denoted by element numbers ending in 06.

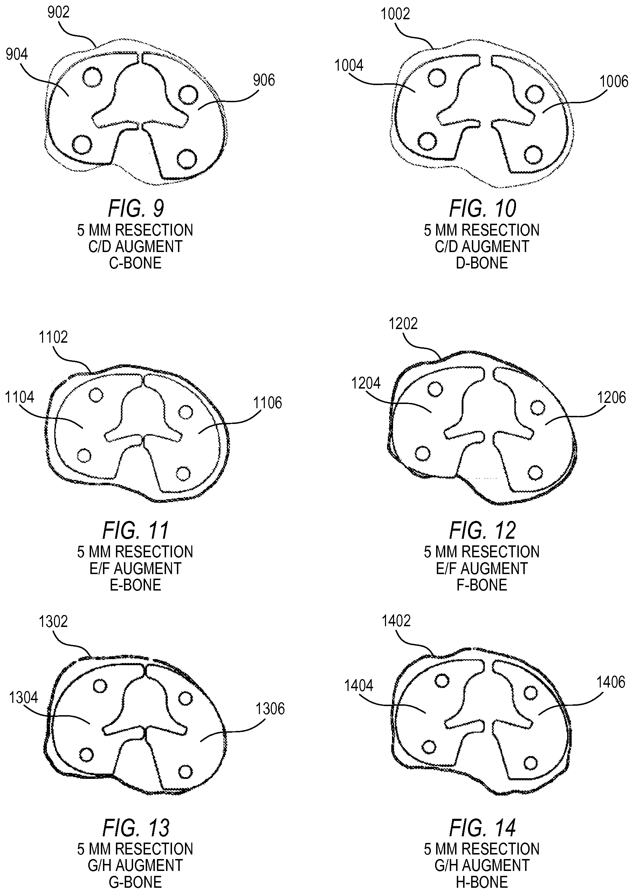

An outline of a particular tibial bone footprint is superimposed onto each tibial augment. The bone footprints in FIGS. 3-14 are denoted by element numbers ending in 02. In general, a practitioner can measure the size and/or shape of a particular bone of a patient, compare the measured bone size and/or shape to several specified sizes, and select one of the specified sizes as best representing the size and/or shape of the bone. An established convention for tibial measurements uses six specified bone sizes, denoted as C, D, E, F, G, and H, although other conventions may be used. Each footprint shown in FIGS. 3-14 is intended to represent an average bone size and shape for a particular specified bone size. For example, an average D-bone may have the footprint shown in FIGS. 4 and 10, but an actual bone classified as D-bone may be a little larger or a little smaller than the average, or may be shaped slightly differently than the average. In this example system, the six specified bone sizes of C, D, E, F, G, and H are addressed by three discrete augment sizes, denoted as C/D, E/F, and G/H. More or fewer discrete augment sizes may also be used.

In seven of the twelve examples shown in FIGS. 3-14, the footprint of the distal side of the tapered augment fits entirely within the footprint of the tibia bone. In other words, for these seven examples, there is no overhang of the tibial augment, for an average bone size. The other five of the twelve examples show small regions around the periphery where there is a small overhang, mostly at the lateral posterior portion of the augment, and at the larger of the two resection values. For each of the twelve examples, having a taper around the periphery reduces the footprint of the tibial augment, especially compared with a comparable tibial augment that has no taper. In many of the twelve examples, having a taper that can vary on at least a portion of the tibial augment can further reduce the footprint of the tibial augment, especially in the lateral posterior portion of the tibial augment. In some examples, the taper in the lateral posterior portion of the tibial augment may be larger than in other portions of the tibial augment.

The above Detailed Description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, system, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. The scope of the invention is defined by the appended claims.

## Claims

1. A device for augmenting a tibial stem for a human knee, the tibial stem coupled to a distal side of a tibial platform (120), a proximal side of the tibial platform couplable to a surface for articulation with a femoral component, the tibial platform having a medial side (124) and a lateral side (122), the device comprising:
a tibial augment (100) configured to couple to the tibial platform (120) proximate a distal side of the tibial platform, the tibial augment including a medial side (106) for attachment to the medial side of the tibial platform, the medual side of the tibial augment having a medial taper (118) on at least a portion of its periphery extending inward in a distal direction, and a lateral side (104) for attachment to the lateral side of the tibial platform, the lateral side of the tibial augment having a lateral taper (116) on at least a portion of its periphery extending inward in a distal direction;
wherein at least a portion of the medial taper is different from at least a portion of the lateral taper; and
wherein the medial taper (118) is constant over the periphery of the medial side (106) of the tibial augment; and
wherein the lateral taper (116) is constant over the periphery of the lateral side (104) of the tibial augment.

2. The device of claim 1, wherein the medial taper (118) is between thirteen degrees and eighteen degrees, inclusive.

3. The device of any one of claims 1-2, wherein the lateral taper (116) is between ten degrees and eighteen degrees, inclusive.

4. The device of any one of claims 1-3, wherein the tibial augment (100) is a tibial augment implant configured to be fixedly attachable to the tibial platform (120).

5. The device of any one of claims 1-4, wherein the medial side (106) and the lateral side (104) of the tibial augment (100) are disposable on opposite sides of the tibial stem.

6. The device of any one of claims 1-5, wherein the tibial augment (200) includes a plurality of augment holes (240, 242, 244, 246) extending therethrough, the plurality of augment holes configured to align with a plurality of holes of the tibial platform.

7. The device of any one of claims 1-6, wherein the medial side (106) and the lateral side (104) of the tibial augment are discrete elements.

8. The device of any one of claims 1-7 wherein the tibial augment (100, 200) includes a periphery sized and shaped to match a periphery size and shape of the tibial platform (120).

9. The device of any one of claims 1-8, wherein the tibial augment (100, 200) includes planar proximal and distal surfaces.

10. The device of any one of claims 1-9, wherein the medial taper (118) and the lateral taper (116) are configured to match an existing bone profile.

11. The device of any one of claims 1-10, wherein the tibial augment (100, 200) is part of a system that includes a plurality of differently-sized augments.

## Patentansprüche

1. Vorrichtung zur Augmentation eines Tibiaschaftes für ein menschliches Knie, wobei der Tibiaschaft an eine distale Seite einer tibialen Plattform (120) gekoppelt ist, wobei eine proximale Seite der tibialen Plattform an eine Oberfläche zur Artikulation mit einer femoralen Komponente koppelbar ist, wobei die tibiale Plattform eine mediale Seite (124) und eine laterale Seite (122) hat, wobei die Vorrichtung Folgendes aufweist:
eine Tibiaagumentation (100), die gestaltet ist, um an die tibiale Plattform (120) nahe einer distalen Seite der tibialen Plattfom anzukoppeln, wobei die Tibiaaugmentation eine mediale Seite (106) zur Anbringung an der medialen Seite der tibialen Plattform umfasst, wobei die mediale Seite der Tibiaaugmentation eine sich nach nach innen in einer distalen Richtung erstreckende, mediale Verjüngung (118) an mindestens einem Abschnitt ihres Umfangs und eine laterale Seite (104) zur Anbringung an der lateralen Seite der tibialen Plattform hat, wobei die laterale Seite der Tibiaaugmentation eine sich nach nach innen in einer distalen Richtung erstreckende, laterale Verjüngung (116) an mindestens einen Abschnitt ihres Umfangs hat,
wobei mindestens ein Abschnitt der medialen Verjüngung sich von mindestens einem Abschnitt der lateralen Verjüngung unterscheidet und
wobei die mediale Verjüngung (118) konstant über den Umfang der medialen Seite (106) der Tibiaaugmentation ist und
wobei die laterale Verjüngung (116) konstant über den Umfang der lateralen Seite (104) der Tibiaaugmentation ist.

2. Vorrichtung nach Anspruch 1, wobei die mediale Verjüngung (118) zwischen einschließlich dreizehn Grad und achtzehn Grad beträgt.

3. Vorrichtung nach einem der Ansprüche 1-2, wobei die laterale Verjüngung (116) zwischen einschließlich zehn Grad und achtzehn Grad beträgt.

4. Vorrichtung nach einem der Ansprüche 1-3, wobei die Tibiaaugmention (100) ein Tibiaaugmentionsimplantat ist, das gestaltet ist, um fest an der tibialen Plattform (120) anbringbar zu sein.

5. Vorrichtung nach einem der Ansprüche 1-4, wobei die mediale Seite (106) und die laterale Seite (104) der Tibiaaugmentation (100) an gegenüberliegenden Seiten des Tibiaschafts anordenbar sind.

6. Vorrichtung nach einem der Ansprüche 1-5, wobei die Tibiaaugmentation (200) eine Vielzahl von sich dadurch erstreckenden Augmentationslöchern (240, 242, 244, 246) umfasst, wobei die Vielzahl von Augmentationslöchern gestaltet ist, um mit einer Vielzahl von Löchern der tibialen Plattform zu fluchten.

7. Vorrichtung nach einem der Ansprüche 1-6, wobei die mediale Seite (106) und die laterale Seite (104) der Tibiaaugmentation diskrete Elemente sind.

8. Vorrichtung nach einem der Ansprüche 1-7, wobei die Tibiaaugmentation (100, 200) einen Umfang umfasst, der so bemessen und geformt ist, dass er mit einer Umfangsgröße und -form der tibialen Plattform (120) übereinstimmt.

9. Vorrichtung nach einem der Ansprüche 1-8, wobei die Tibiaaugmentation (100, 200) ebene proximale und distale Oberflächen umfasst.

10. Vorrichtung nach einem der Ansprüche 1-9, wobei die mediale Verjüngung (118) und die laterale Verjüngung (116) gestaltet sind, um zu einem vorhandenen Knochenprofil zu passen.

11. Vorrichtung nach einem der Ansprüche 1-10, wobei die Tibiaaugmention (100, 200) Teil eines Systems ist, das eine Vielzahl von Augmentationen unterschiedlicher Größe umfasst.

## Revendications

1. Dispositif permettant d'augmenter une tige tibiale d'un genou humain, la tige tibiale étant couplée à un côté distal d'une plate-forme tibiale (120), un côté proximal de la plate-forme tibiale pouvant être couplé à une surface à des fins d'articulation avec un composant fémoral, la plate-forme tibiale comportant un côté interne (124) et un côté latéral (122), le dispositif comprenant :
une augmentation tibiale (100) conçue pour être couplée à la plate-forme tibiale (120) à proximité d'un côté distal de la plate-forme tibiale, l'augmentation tibiale comprenant un côté interne (106) permettant une fixation au côté interne de la plate-forme tibiale, le côté interne de l'augmentation tibiale ayant une conicité interne (118) sur au moins une partie de sa périphérie s'étendant vers l'intérieur dans un sens distal, et un côté latéral (104) permettant une fixation au côté latéral de la plate-forme tibiale, le côté latéral de l'augmentation tibiale ayant une conicité latérale (116) sur au moins une partie de sa périphérie s'étendant vers l'intérieur dans un sens distal ;
dans lequel au moins une partie de la conicité interne est différente d'au moins une partie de la conicité latérale ; et
dans lequel la conicité interne (118) est constante sur la périphérie du côté interne (106) de l'augmentation tibiale ; et
dans lequel la conicité latérale (116) est constante sur la périphérie du côté latéral (104) de l'augmentation tibiale.

2. Dispositif selon la revendication 1, dans lequel la conicité interne (118) est comprise entre treize degrés et dix-huit degrés, inclus.

3. Dispositif selon l'une quelconque des revendications 1 et 2, dans lequel la conicité latérale (116) est comprise entre dix degrés et dix-huit degrés, inclus.

4. Dispositif selon l'une quelconque des revendications 1 à 3, dans lequel l'augmentation tibiale (100) est un implant d'augmentation tibiale conçu pour être fixé à demeure à la plate-forme tibiale (120).

5. Dispositif selon l'une quelconque des revendications 1 à 4, dans lequel le côté interne (106) et le côté latéral (104) de l'augmentation tibiale (100) peuvent être disposés sur des côtés opposés de la tige tibiale.

6. Dispositif selon l'une quelconque des revendications 1 à 5, dans lequel l'augmentation tibiale (200) comprend une pluralité de trous d'augmentation (240, 242, 244, 246) s'étendant à travers cette dernière, la pluralité de trous d'augmentation étant conçue pour s'aligner avec une pluralité de trous de la plate-forme tibiale.

7. Dispositif selon l'une quelconque des revendications 1 à 6, dans lequel le côté interne (106) et le côté latéral (104) de l'augmentation tibiale sont des éléments discrets.

8. Dispositif selon l'une quelconque des revendications 1 à 7, dans lequel l'augmentation tibiale (100, 200) comprend une périphérie dimensionnée et formée de façon à correspondre à une taille et une forme de périphérie de la plate-forme tibiale (120).

9. Dispositif selon l'une quelconque des revendications 1 à 8, dans lequel l'augmentation tibiale (100, 200) comprend des surfaces proximale et distale planes.

10. Dispositif selon l'une quelconque des revendications 1 à 9, dans lequel la conicité interne (118) et la conicité latérale (116) sont conçues pour correspondre à un profil osseux existant.

11. Dispositif selon l'une quelconque des revendications 1 à 10, dans lequel l'augmentation tibiale (100, 200) fait partie d'un système qui comprend une pluralité d'augmentations de tailles différentes.
